# EUROPEAN PATENT SPECIFICATION

(11) **EP 4 333 922 B1**
(45) Date of publication and mention of the grant of the patent: **03.09.2025**
(21) Application number: 22730669.3
(22) Date of filing: 04.05.2022
(51) Int. Cl.: A61L 31/02, A61L 31/10, A61L 31/16

(54) **NEW DEVICES FOR USE FOR TREATING CEREBRAL ANEURYSMS**
NEUE VORRICHTUNGEN ZUR BEHANDLUNG VON ZEREBRALEN ANEURYSMEN
NOUVEAUX DISPOSITIFS À UTILISER POUR LE TRAITEMENT DES ANÉVRISMES CÉRÉBRAUX

(30) Priority: 04.05.2021 EP 21305573
(43) Date of publication of application: 13.03.2024
(73) Proprietor: Institut national de la santé et de la recherche médicale (INSERM), 75013 Paris (FR); Commissariat à l'Energie Atomique et aux Energies Alternatives, 75015 Paris (FR); Conservatoire National des Arts et des Métiers, 75141 Paris Cedex 03 (FR); Chu De Limoges, 87042 Limoges (FR); Université Paris Cité, 75006 Paris (FR); Universite Paris Nord, 93430 Villetaneuse (FR); Centre National de la Recherche Scientifique, 75016 Paris (FR); Université de Limoges, 87000 Limoges (FR)
(72) Inventor: ROUCHAUD, Aymeric, 87060 LIMOGES CEDEX (FR); CHAUBET, Frédéric, 93430 VILLETANEUSE (FR); DENIAU, Guy, 91191 GIF SUR YVETTE CEDEX (FR); HAUQUIER, Fanny, 91191 GIF SUR YVETTE CEDEX (FR); SZATMARY, Zoltan, 87060 LIMOGES CEDEX (FR); MAIRE, Murielle, 93430 VILLETANEUSE (FR)
(74) Representative: Icosa
(86) International application number: PCT/EP2022/061998
(87) International publication number: WO 2022/233944

(56) References cited:
- WO-A1-2008/143933
- WO-A1-2019/210414
- WO-A2-03/065881
- US-A1- 2012 231 043

## Description

The present invention concerns the treatment of as cerebral aneurysms.

Aneurysms are outward bulging of blood vessels and are caused by a localized, abnormal, weakness of the blood vessels walls. This is generally due to the scarcity of the cells on the blood vessels walls, leading to the decay of the extracellular matrix which can't withstand the pressure. Weakened walls can suddenly leak, which can lead to dramatic consequences.

Current treatments aim at improving the healing of the aneurysm opening by occluding the aneurysm pocket. For this, commercial intrasaccular devices are used such as platinum coils and Endosaccular Flow Disrupter such as WEB (Woven EndoBridge, self-expanding intra expandable device), made of Nitinol.

Vascular disorders are generally treated by stents coated with active ingredients. However, the treatment of aneurysms is highly specific: it differs mainly in that a pro-aggregation/pro-thrombotic effect is desired to enhance proliferation of the cells on the walls of the endoprothesis, whereas an anti-coagulation effect is generally desired in the treatment of vascular conditions, such as atherosclerosis.

Additionally, intravascular devices used for aneurysms differ from vascular stents in that they have a distinctive structure/geometry and are made of different materials.

Further, coils and webs used in cerebral aneurysms aim at healing by cicatrization, whereas stents used in vascular conditions aim at endothelialization.

Also, the vascular stents are generally coated with active ingredients that are released in the body, which is neither desired nor required in the case of aneurysms.

In any case, the current treatments of aneurysms with endovascular devices are generally ineffective in 30% of cases due to a lack of cells to promote healing of the aneurysm as the material does not foster the cicatrization.

There is therefore a need to provide an improved device to enhance cell proliferation and healing for treating aneurysms.

International patent application WO03/065881 discloses medical devices implanted in vessels or hollow organs, said devices being coated in order to promote progenitor endothelial cells to adhere, grow and differentiate on the surface of the implanted device to form a functional endothelium.

Fucoidans are sulphated polysaccharides that can be found *inter alia* in the extracellular matrix of brown marine algae.

Originally named fucoidin in 1913 by Kylin, it was renamed fucoidan by IUPAC. The fucoidans, mostly composed of sulphated fucose, belong to the fucan family which also includes ascophyllans (branched polyuronics) and sargassans (compounds of galactose, fucose and uronic acid). Fucoidans can be found in more of 70 species of marine algae.

Fucoidans have been reported to exhibit various biological activities, such as pro/antithrombotic, angiogenic, anti-cancer, anti-inflammatory and anti-oxidant activities.

Chollet et al (Thesis of Université Paris 13, 2017 « Fractions polysaccharidiques bioactives extraites de I'algue brune Ascophyllum nodosum pour le diagnostic et la thérapeutique de pathologies cardiovasculaires : caractérisation et optimisations du procédé ») disclosed the extraction and purification of fucoidans, their affinity for P-selectin which is used for thrombose imaging. Specifically, it was investigated whether atherosclerosis could be diagnosed with fucoidans-grafted gold chips.

It has now been discovered that an improved aneurysms endoprothesis grafted with fucoidans exhibit improved healing properties.

The present invention thus provides an aneurysms endoprothesis grafted with fucoidans.

According to an object, the present invention provides an aneurysms endoprothesis comprising
a metallic support and
a biologically active film covalently grafted on said metallic support,

characterized in that said film comprises one or more polyaryl chains having a proximal end that is covalently grafted to said metallic support, and
at least one distal end that is covalently bound with a Fucoidan terminal group through a terminal functional group comprising the following group of formula (I):

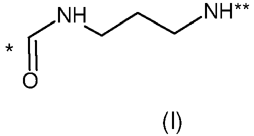

Where * represents the covalent attachment with a polyaryl chain ; and
** represents the covalent attachment with the Fucoidan terminal group.

As used therein,the term « biologically active » refers to the activity exerted by the fucoidan molecules that are grafted to the endoprothesis.

Said activity includes in particular the healing effect by increasing the cell proliferation.

According to the invention, the Fucoidan groups are covalently attached to the metallic support through the polyaryl chains, and as such are not released upon their implantation.

According to an embodiment, the film comprises at least one one chain of formula (II) :

-***Linker**-Fucoidan (II)

Where in formula (II) :
Fucoidan represents a fucoidan polysaccharide ;
Linker represents a linear or branched polyaryl chain;
*** represents the covalent attachment of the Linker group with the metallic support and
** represents the covalent attachment of the Linker group with the Fucoidan terminal group.

Said Linker has a terminal (proximal) end that is covalently grafted on the metallic support and one or more terminal (distal) ends that are covalenly bound with the Fucoidan group. Typically, said Linker comprises at its terminal distal end the terminal functional group comprising the group of formula (I) as defined above for covalently attachment with the Fucoidan.

According to an embodiment, said Linker may be represented by Formula (III) : 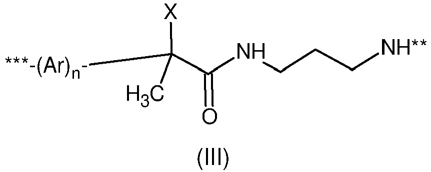
Where *** and ** are defined as above ;
X represents H or -(Ar)ₙ- and
-(Ar)ₙ- represents a linear or branched repetition of n aryl units, with n comprised between 1 and 10 ;
wherein
Ar represents a 5 to 10 membered aryl group, this aryl group possibly comprising one or more aromatic or heteroaromatic rings, condensed or not, optionally mono- or polysubstituted;
Said optional substituents being chosen from alkyl, haloalkyl such as chloro- or fluoroalkyl, alkoxy, haloalkoxy, nitro, cyano, aldehyde, hydroxyl, ketone, carbonyl type, carboxyl, ester, ether, amine, amide, nitrile, sulfonic, halogen atoms.

As used herein, "Aryl" means an aromatic monocyclic or multicyclic ring system of about 6 to about 14 carbon atoms, preferably of about 6 to about 10 carbon atoms. Exemplary aryl groups include phenyl or naphthyl, or phenyl substituted or naphthyl substituted.

"Heteroaryl" means an aromatic monocyclic or multicyclic ring system of about 5 to about 14 carbon atoms, preferably about 5 to about 10 carbon atoms, in which one or more of the carbon atoms in the ring system is/are hetero element(s) other than carbon, for example nitrogen, oxygen or sulfur. Preferred ring sizes of rings of the ring system include about 5 to about 6 ring atoms. Exemplary heteroaryl and substituted heteroaryl groups include pyrazinyl, thienyl, isothiazolyl, oxazolyl, pyrazolyl, furazanyl, pyrrolyl, 1,2,4-thiadiazolyl, pyridazinyl, quinoxalinyl, phthalazinyl, imidazo[1,2-a]pyridine, imidazo[2,1-b]thiazolyl, benzofurazanyl, azaindolyl, benzimidazolyl, benzothienyl, thienopyridyl, thienopyrimidyl, pyrrolopyridyl, imidazopyridyl, benzoazaindole, 1,2,4-triazinyl, benzthiazolyl, furanyl, imidazolyl, indolyl, indolizinyl, isoxazolyl, isoquinolinyl, isothiazolyl, oxadiazolyl, pyrazinyl, pyridazinyl, pyrazolyl, pyridyl, pyrimidinyl, pyrrolyl, quinazolinyl, quinolinyl, 1,3,4-thiadiazolyl, thiazolyl, thienyl and triazolyl. Preferred heteroaryl groups include pyrazinyl, thienyl, pyridyl, pyrimidinyl, isoxazolyl and isothiazolyl.

According to an embodiment, Ar represents a phenyl group optionally substituted by at least one substituent chosen from alkyl, haloalkyl, alkoxy, haloalkoxy, nitro, cyano, aldehyde, hydroxyl, ketone, carbonyl, carboxyl, ester, ether, amine, amide, nitrile, sulfonic and/or halogen atoms; more particularly nitro.

Particularly preferably, the aryl group is a phenyl group, optionally bearing one or more substituents, whereby the polyarylene film which is grafted is a film of a polyphenylene. Even more specifically, the aryl group is a phenyl group substituted by a nitro group, in which case the film formed is a film of a poly (nitrophenylene).

More particularly, Ar (also referred to as the aryl unit in the polyaryl chain) is an optionally substituted nitrophenyl unit, such as an optionally substituted 4-nitrophenyl, of formula (IV) : 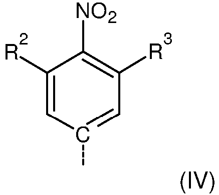 Where
--- represents a covalent bond with the metallic support;
R², R³ identical or different independently represent H or a covalent bond with another Ar unit or with the terminal functional group as defined above, provided at least one of R², R³ is a covalent bond.

Accordingly, where the aryl unit is a group of formula (IV), in the proximal terminal unit,typically represents the covalent bond with the metallic support and in the one or more distal terminal units, one or more of R², R³ typically represents the covalent bond with the functional terminal group as defined above.

Said Fucoidan may be a synthetic or natural polysaccharide.

In particular it may be extracted from brown algae, such as *Chorda filum*, *Ascophyllum nodosum, Fucus vesiculosus, Fucus evanescens, Laminaria saccharina, Fucus serratus L. and Cladosiphon okamuranus.*

It can also be extracted from marine invertibrate animals such as *Ludwigothuria grisea*; *Strongylocentrotus droebachiensis* and *Strongylocentrotus franciscanus.*

The structure of fucoidans from different algae has been extensively studied during the last century. It depends on both the species and the parameters (ecosystem, season, location), as well as the extraction method.

The first structure was highlighted in 1950 by Conchy and Percival from a fucoidan from *Fucus vesiculosus.* The main chain consists of a linear skeleton of α (1 → 2) -L-fucose with a substitution of the sulfate function in C4 but also occasional bonds α (1 → 4). In 1993 the model was corrected by Patankar et al. : a linear chain of fucoses linked in α (1 → 3) with a C4 sulfate and a few branches in C4 or C2, in the end about one modification for two or three fucose residues within the chain. Subsequently, studies have shown the existence of recurrent units for purified fractions from different species of algae. The structures are linear α (1->3) -L-fucose skeletons with alternately α (1->4) bonds, the sulphate groups being mainly found on positions O-2 and O-4. Fucoidans extracted from marine animals exhibit more regular structures with little or no branching. Representative structures of fucoidans are depicted below: 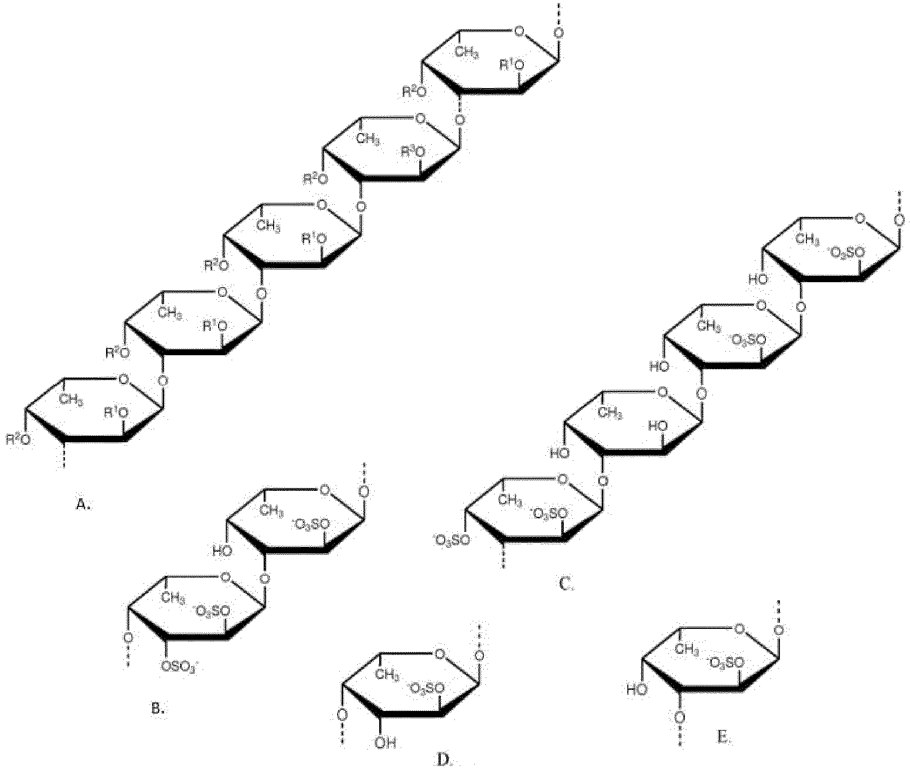
R¹ = SO₃⁻ ou, H ou COCH₃
R²= SO₃⁻ ou H

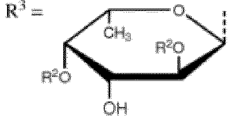

### (A) Chorda filum; (B) Ascophyllum nodosum, Fucus vesiculosus, Fucus evanescens ; (C) Ludwigothuria grisea; (D) Strongylocentrotus droebachiensis ; (E) Strongylocentrotus franciscanus

Or the pharmaceutically acceptable salts thereof.

According to an embodiment, the fucoidan is an extract from *Ascophyllum nodosum*, in particular a depolymerized fraction thereof.

Typically, the fucoidan is a mixture having an average (weight) molar weight comprised between 5000 and 30000 g/mol (kDa), typically between 5 and 15 kDa.

Suitable fucoidans for the present invention may be commercially available. A suitable fucoidan composition may be purchased from Algues et Mer under the trade name Ascophyscient^{®}. Ascophyscient^{®} is a low molecular weight depolymerized fraction extracted from *Ascophyllum nodosum.*

According to an embodiment, said fucoidan is a compound comprising the repetition of one or more units of formula (V) :

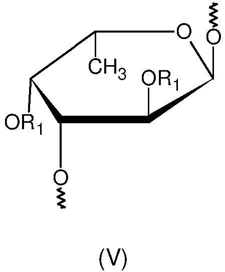

Where R₁ is chosen from H ; SO₃X, where X represents H or a cation, typically an alkali metal, such as Na⁺ ; or a lateral sugar chosen from xylose, glucose, galactose, mannose, or a glucoronic acid ;
provided at least one of R₁ is SO₃X,
or a mixture thereof,
Or the pharmaceutically acceptable salts thereof.

The compounds of formula (V) can be provided in the form of a free base or in the form of addition salts with acids, which also form part of the invention.

These salts are advantageously prepared with pharmaceutically acceptable acids, but salts with other acids, useful for example for the purification or for the isolation of the compounds of formula (V), also form part of the invention.

According to an embodiment, the endoprothesis may be any of the devices that are implanted for healing aneurysms, such as cerebral aneurysms. Typical endoprothesis are coils or WEBs (Woven EndoBridge, self-expanding intra expandable device). They may be typically made of platinum, nickel, titane, and alloys thereof, such as Nitinol (nickel/titane).

According to another object, the present invention concerns the process of manufacture of the endoprothesis according to the invention.

Said process comprises the following steps :
- Functionalization of the fucoidan so as to introduce a metacrylic acid terminal group, and
- Grafting of said functionalized fucoidan with the metallic support by means of an aryldiazonium salt.

According to an embodiment, the functionalization step comprises :
- Reductive amination of the Fucoidan ; and
- Methacrylation of said reduced fucoidan.

Reductive amination consists in grafting a primary amine at the end of the polysaccharide chain onto the aldehyde function of the fucoidan.

According to an embodiment, diaminopropane may be chosen as a suitable primary amine. Typically, the reductive amination step may be conducted by reacting the fucoidan with said primary amine, followed by reacting the reactional mixture with dimethylborane (C₂H₇B). The reaction with the primary amine can be conducted in protic conditions, typically in an aqueous acidic solution, such as acetic acid in water. The reaction may be conducted under heating at a temperature comprised between the room temperature and the reflux temperature of the reactional mixture.

Typically, dimethylborane may be used as a diluted aqueous solution.

Typically, the metacrylic acid group introduced at the fucoidan is formula:

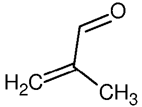

The methacrylation step comprises reacting the aminated fucoidan with methacrylic anhydride. This reaction may be typically conducted in aprotic conditions, such as in DMF

The functionalized fucoidan that is so obtained in then grafted onto the metallic support. The grafting may be achieved by using as an anchor an aryldiazonium salt of formula (VI):

Y⁻ / N₂⁺-Ar (VI)

Where Y⁻ represents a counter ion and Ar is defined as in formula (III) above.

Said aryldiazonium salt may be a nitrobenzodiazonium salt of formula (VII):

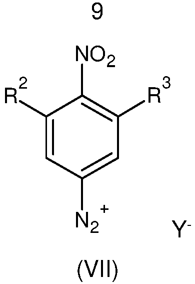

Where R³ and R² are defined as in formula (IV), and Y⁻ represents a counter ion.

The counter ion may be an inorganic anion such as a halide ion such as a bromide (Br), iodide (I⁻) or chloride (Cl⁻) ion, a tetrahaloborate ion such as a tetrafluoroborate ion (BF₄⁻), a hydrogen sulphate ion (HSO₄⁻), a dihydrogen phosphate ion (H₂PO₄⁻), a nitrate ion (NO₃⁻) or a chlorate ion (ClO₃⁻). However, it can also be an organic anion such as an acetate ion (CH₃CO₂⁻) or a formate ion (HCO₂).

Suitable aryldiazonium salts may include phenyldiazonium tetrafluoroborate, 4-methylphenyldiazonium tetrafluoroborate, 4-nitrophenyldiazonium tetrafluorobrate, 4-carboxyphenyldiazonium tetrafluoroborate, tetrafluoroborate of 4-carboxyphenyldiazonium tetrafluoroborate. 4-carboxymethylphenyl-diazonium, 4-chloromethylphenyldiazonium tetrafluoroborate, 4-bromophenyldiazonium tetrafluoroborate, tetrafluoroborate 4-diethylaminophenyldiazonium, 4-cyanophenyldiazonium tetrafluoroborate, 4-aminophenyldiazonium chloride, 4-aminomethylphenyldiazonium chloride, 4-methoxyphenyldiazonium tetrafluoroborate, tetrafluoroborate 4-heptadecylfluorooctylphenyldiazonium and 4-acetamido-phenyldiazonium tetrafluoroborate.

Specifically, the counter ion may be a tetrafluoroborate ion (BF₄⁻), so that the aryldiazonium salt is NBDT (nitrobenzodiazonium tetrafluoroborate):

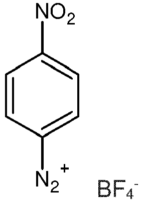

The grafting onto the metallic support may be typically achieved by reduction of the aryldiazonium salt leading to aryldiazonium radicals. This reduction may be typically conducted by either of the two following alternatives procedures:
According to a first alternative, grafting is conducted by electrochemical reduction of the aryldiazonium salt, including electrografting.

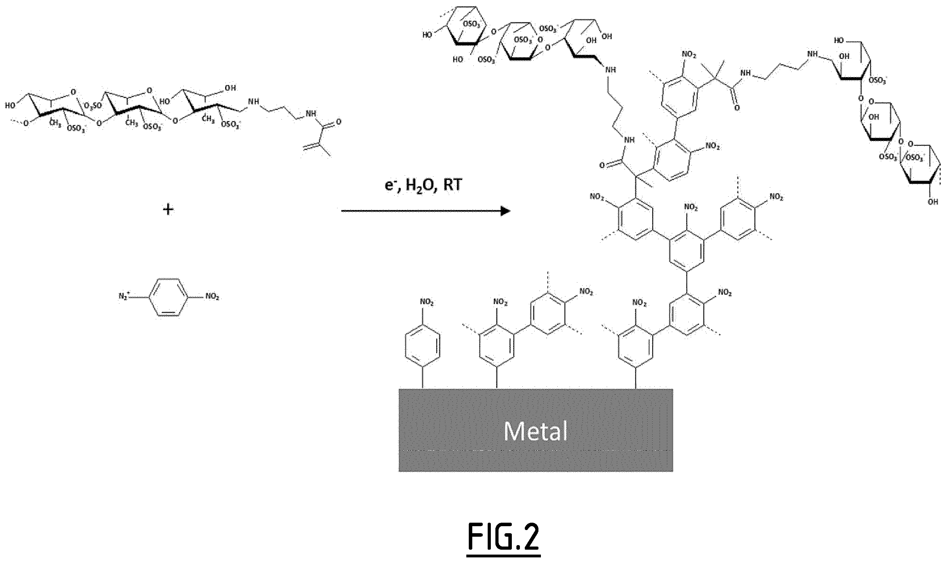

Electrografting is an electrochemical method for surface modification, that can be implemented by application or adaptation of the method disclosed by Belanger et al in Chemical Society Reviews, issue 7, January 2011. It may be commercially available under the tradename eG^{®} :
Cyclic voltammetry is used to reduce the diazonium salt, causing the formation of radicals. Some radicals graft on the metallic support and some radicals react with the methacrylated Fucoidan. (Figure 2B).

According a second alternative, grafting is conducted in a chemically induced reduction of the aryldiazonium salt, in the presence of ascorbic acid as a reducing agent. According to this alternative, the metallic support is contacted with a solution of ascorbic acid, the diazonium salt and the functionalized fucoidan. The mechanism of grafting relies on a key step, which is the formation of aryl radicals from the ascorbic acid-induced reduction of the diazonium salt in solution. Indeed, those radicals have a double role. They can graft onto the substrate and form a primer-layer (rich in aromatic rings) essential for the subsequent grafting of functionalized fucoidan; meanwhile they initiate the radical polymerization of the vinyl cgain of the functionalized fucoidan in solution. Finally, the growing radical polymer chains eventually graft by a "grafting to" pathway on the aromatic rings present on the surface to form a grafted polymer films on the substrate.

This may be conducted by application or adaptation of the Graftfast^{®} procedure.

In the process above, starting compounds and reactants, unless otherwise indicated, are commercially available or described in literature, or can be prepared according to methods described in literature or known to one of skill in the art.

Accordig to a further object, the present invention concerns an endoprothesis as defined above for its use for the treatment and/or prevention of aneurysms, such as cerebral aneurysms.

According to an embodiment, the endoprothesis of the invention is suitable for preventing the recidivism of aneurysms.

### Figures:

Figure 1 represents the 1H NMR spectrum (D2O, 99.8%) of (B) methacrylated fucoidan with the characteristic peaks, and (A) the precursor thereof according to example 1.
Figure 2 illustrates the synthetic pathway for the (A) Grafting of diazonium salts (NBDT: R = NO₂) by electrografting and (B) polymerization of a representative methacrylated fucoidan.
Figure 3 illustrates the XPS spectra from 162 to 174 eV evidenced S2p electrons on a representative fucoidan grafted sample with electrografting (Incident energy = 40 eV).
Figure 4 shows the EDS Images of commercial platinum coil grafted with the fucoidan according to example 1. The bottom row is a magnification of the same area of the sample. A scratch on the polymer layer is evidenced on the right image.
Figure 5 shows the sections of aneurysms explanted 1 month after coiling from NZ rabbits. Coils were either A. bare platinum (untreated), B. dextran covered, or C. fucoidan covered. Sections were stained in Masson's trichrome (x100 magnification). *aw*: aneurysm wall; cs: coil scar; the grey line symbolizes the inner limit of aneurysm wall inside the aneurysm cavity.
Figure 6A and 6B illustrate the healing histological index for aneurysms treated with bare coils or coils with fucoidan.

The following examples are illustrative of the present invention.

### EXAMPLES

### Example 1: Preparation of the polysaccharides for grafting onto metallic surfaces

In order to graft polysaccharides on metals, two processes are considered : Graftfast^{®} and electrografting such as eG^{®}, requiring in both cases the introduction of a methacrylate chemical group at the reducing end of the polysaccharide chain In the presence of a diazonium salt the radical polymerization of the modified polysaccharides occurs forming a polymer layer at the surface of metals .

The modification of the polysaccharide was performed in two steps :
1) The first step consists in grafting a primary amine at the end of the chain onto the aldehyde function of the polysaccharide. An illustrative exemple is provided below with representative fucoidans.

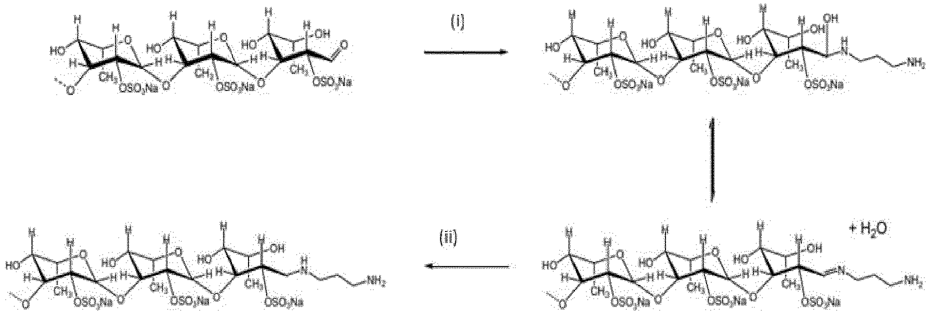

### Reductive amination of fucoidan. (i) diaminopropane, water/acetic acid, 3h, 90°C; (ii) dimethylborane, water/acetic acid, 3h, 90°C

It is to be understood that although the starting fucoidan is generally represented with a terminal cyclic form, the opened form form which is in equilibrium with said cyclic form has been depicted above as it is locked in this form by the reaction with the diamine.

### Method

In a 10 mL flask, about 600 mg of polysaccharide previously dried overnight under vacuum at 60 ° C is dissolved in a solution prepared with 1.25 mL of diaminopropane 1.5 M, 5 mL of water and 3.75 mL of acetic acid. The flask is heated at 90 ° C for 3 hours. After cooling the flask in an ice bath, add 1.4 mL of 3M dimethylborane solution freshly prepared. The solution is heated for 3h at 90°C. After cooling, the solution is dialyzed (MWCO 1kDa) successively against 0.5 M carbonate buffer at pH9.6 with 1M NaCl (5x1L), a water/ethanol mixture (1:1, v/v) with 0.5 M NaCl (5x1L), and osmotic water (5x1L). The final solution is frozen and lyophilized. The product is obtained as a colorless foam in 50-80% yield. The amount of primary amines introduced per polymer chain evaluated by colorimetric assay with o-phthalaldehyde using bromopropylamine as standard is ranging from 0.7 to 0.95.

2) The second step is the methacrylation of the primary amine introduced in the polymer chain. An exemple is provided below with representative fucoidan.

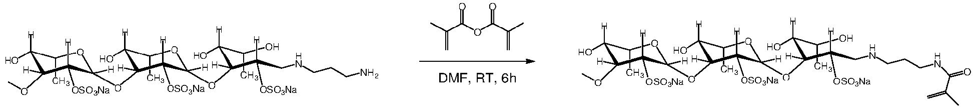

### Methacrylation of the amino fucoidan.

### Method

In a 25 mL flask, about 120 mg of aminated polysaccharide previously dried overnight under vacuum at 60 ° C is dissolved under nitrogen in 5 mL of anhydrous DMF. Then 0.375 mL of 0.1 M methacrylic anhydride in anydrous DMF are added and kept under stirring and nitrogen bubbling for 6h at room temperature. Then, the solution is dialyzed (MWCO 1kDa) successively against 0.5 M carbonate buffer at pH9.6 with 1M NaCl (5x1L), a water/ethanol mixture (1:1, v/v) with 0.5 M NaCl (5x1L), and osmotic water (5x1L). The final solution is frozen and lyophilized. The product is obtained as a colorless foam in 60-92% yield.

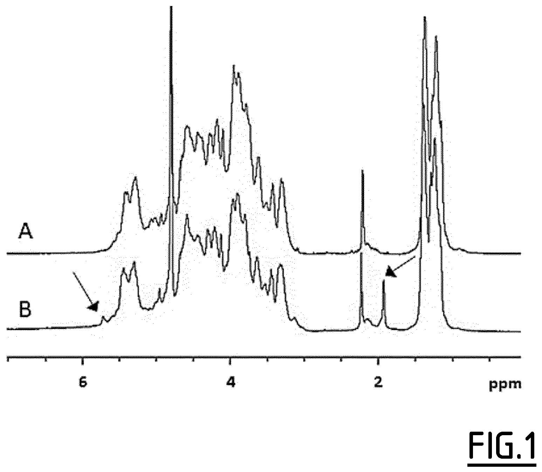

1H-NMR analysis allows to validate the presence of the methacrylic group (see Figure 1).

### Grafting of the methacrylated polysaccharides onto metallic surfaces

Figure 2 presents schematically the grafting of methacrylated fucoidan, as an exemple. With the electrochemical method, the grafting is obtained by electro-initiated radical polymerization using cyclic voltammetry.

The Graftfast^{®} process is a so-called "one-pot" process as the reducing agent is added in the reactional mixture. All the compounds are put into solution from the start. Graftfast^{®} uses ascorbic acid as a reducing agent instead of electrochemistry.

### Electrochemical method

A potential is applied (from + 0.5V to -1.5V) of constant sweep (about 50 mV / s) starting from a potential where no reaction is initiated. The potential starts at 0.5V and decreases to -1.5V passing through 0.2V where the reduction of diazonium salts (NBDT) in solution begins. The potential returns to its initial value. 10 cycles are performed. The experiments are carried out in a glass cell with 3 electrodes: the metal sample (coil) on which the polysaccharide is to be fixed serves as the working electrode, an Ag/AgNO3 electrode used as a reference electrode, and the counter electrode made of platinum.

### Graftfast^{®} method

The reagents are introduced in the following molar ratios NBDT/ascorbic acid/methacrylated polymer: 1/0.1/20-350. The sample (coil) to be grafted is placed in contact with the solution and the mixture is left to act for 30 minutes at room temperature under nitrogen bubbling. After 30 minutes the sample is taken out and washed with osmotic water, ethanol before drying in an oven at 50°C.

### XPS analysis

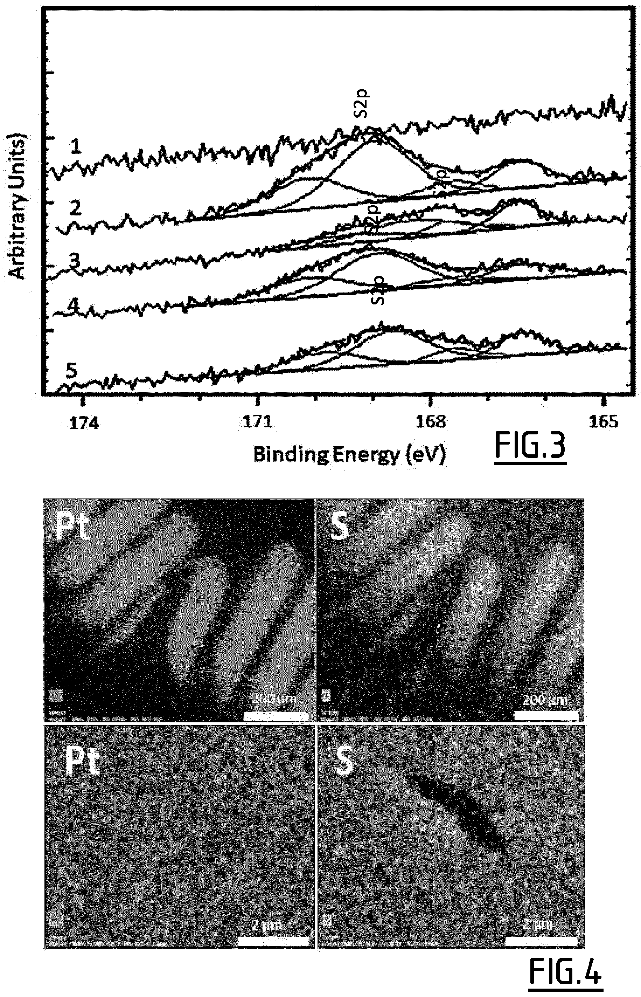

Analyzes were performed on samples grafted with fucoidan using electrografting or graftfast^{®}. In samples, carbon (C), oxygen (O), nitrogen(N), platinum (Pt) and sulfur (S) atoms were evidenced (table 1). Platinum (Pt) was used to calibrate the spectra. The Figure 3 presents the charactristic signals from 2p electrons of sulfur.

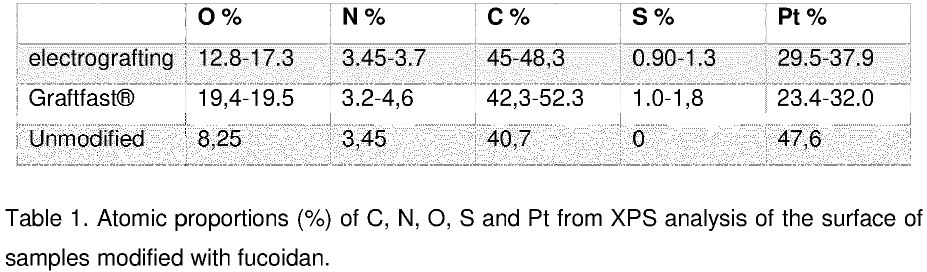

Since only fucoidan provided sulfur, fucoidan is thus grafted to the surface of the sample with both methods.

### EDS analysis

An EDS analysis allowed an elementary mapping of the surface of a sample. The Figure 4 exhibits images of commercial platinum coil grafted with fucoidan using the graftfast method. As expected, a homogeneous distribution of the platinum and sulfur is observed.

### Example 2 : Animal experiments

Type: New Zealand white rabbits
Number: 44 treated rabbits, with 27 harvested aneuryms

The aneurysms are created on New Zealand white rabbits (3.5-4 kg) under general anesthesia by intramuscular injection of ketamine (20 mg/kg) + Xylazine (4.5 mg/kg) + Acepromazine 1% (1 mg/kg). After shaving the cervical area, local anesthesia by subcutaneous injection of 2% lidocaine (Xylocaine^{®}, ^{©}AstraZeneca) is performed followed by a median incision to expose the right Common Carotid Artery (CCA). Following dissection of the artery, an arteriotomy is conducted, and a 5 French introducer (Mini-Plastic Guide 5 Fr. Radiofocus^{®}, Introducer II, Terumo Europe) is advanced retrogradely to 1cm from the ostium of the right CCA. 200 I.U. heparin (Heparin Choay^{®} sodium, 25000 I.U. / 5 mL) is administered by intravenous injection to prevent the formation of per procedural arterial thrombi. Under X-ray scopic control (Mobil C-arm - Siemens Arcadis Varic Model No.08079233 Serial No. 6676 IVK; Munchen, Germany), a 4 French arterial embolectomy catheter (CEM804, Vygon, France)is advanced retrogradely to the origin of the right CCA. At this location, the embolectomy balloon is inflated with a solution containing contrast medium (Hexabrix^{®} 320 mg/mL, Guerbet, Roissy CdG, France) in order to generate a filling chamber. A solution containing 45 I.U. of porcine pancreatic elastase (Classification of the enzyme by IUB System:3.4.21.36; Reference: Code: ES Cat. No. LS002280 Size 1gm; Worthington Biochemical Corporation) is injected into this filling chamber through the introducer, and is left to incubate for 20 minutes. The embolectomy catheter and the introducer are then removed and the carotid is ligated distally.

After suturing the deep muscle planes and the skin plane with a Vicryl 4-0, the rabbit is reinserted into its cage. The development of an aneurysm requires 3 weeks after the surgical creation of the aneurysm. This experimental model provides aneurysms that are histologically similar to human intracranial aneurysms with sizes roughly 5-10 mm larger in diameter and a collar of approximately 4 mm.

Aneurysms were treated 3 weeks after their creation. Under general anesthesia, a 5 F Envoy guide catheter (Codman Neurovascular, Raynham, MA, USA) was navigated from the right common femoral artery into the brachiocephalic trunk. Using the coaxial technique, an Excelsior SL 10 microcatheter (Stryker, Fremont, CA, USA) was advanced into the aneurysm. Either Axium (Medtronic, Irvine, CA) or Optima (BALT, Montmorency, France) Detachable bare platinum coils were used. Aneurysms were embolized with bare platinum coils (control group, n=7) or bare platinum coils covered with dextran (control group, n=6) or bare platinum coils covered with fucoidan (test group, n=14). No anti-platelet agents were given post embolization. The dextran was a witness to show whether fucoidan actually increases the cell proliferation/healing capacity. After embolization control DSA was performed.

4 weeks after embolization a final control DSA was performed followed by euthanasia of the animals by using a lethal injection of embutramide ensued by tissue harvest. Median sternotomy and pericardiotomy were executed. Access to the left ventricle was secured by direct puncture with a 20-gauge catheter. A small cut was done at the right atrial appendage. With pressure pump, heparinized saline (100U/ml) was constantly perfused into the catheter, until the effluent from the right appendage was light pink. The coil aneurysm was then collected and submerged into phosphate buffer saline (PBS). The parent artery was cut longitudinally to visualize the neck orifice for gross inspection to assess the tissue growth at the neck. After photography, the sample was fixed in 2% glutaraldehyde (G5882, Merck, Germany) for 24 hours for further whole tissue mount staining.

A blinded reader with experience estimated aneurysm occlusion and packing density.

### Preparation of sections for histology

Treated tissues were dehydrated in a succession of ethanol baths (12 baths of 24h - 3 at 70%, 3 at 80%, 3 at 95% and 3 at 100%) and embedded in a poly-(methyl-methacrylate) PMMA resin (6 mL methyl methacrylate, 3,5 mL butyl methacrylate, 500 µL methyl benzoate, 120 µL polyethylene glycol 400 (PEG 400), 80 mg Luperox^{®} A75 (benzoyl peroxide) and 60 µL N,N-dimethyl-p-toluidine), 24 hours at 4°C, under neutral atmosphere (nitrogen). Before embedding, dehydrated aneurysms were infused 72 h in a MMA I solution containing the same quantities as above of methyl methacrylate, butyl methacrylate, methyl benzoate and PEG 400, then infused 24 h in a MMA II solution containing MMA I reagents and 40 mg of benzoyl peroxide.

PMMA blocks were then sectioned longitudinally (8 µm section) in a microtome (RM2245, Leica) equipped with a tungsten carbide blade. The sections were deposed on ethanol on Superfrost Microscope Slides (Superfrost Plus^{®} Gold, Thermo Scientific, USA) and let dry 24 h.

### Staining and acquisition for analysis

Slides were stained in hematoxylin-eosin-safran and Masson trichrome. Immunohistochemistry was also performed using EnVision+ Kit (EnVision+ System-HRP anti-mouse, K4001, Dako), and antibodies targeting CD31 (JC70A clone, M0823, Dako), RAM-11 (RAM-11 clone, M0633, Dako) and alpha-SMA (1A4 clone, M0851, Dako).

The slides were scanned on a NanoZoomer RS2.0 (Hamamatsu Photonics, France) and studied with the NDPView software. Images were reviewed by blinded observers.

### Results

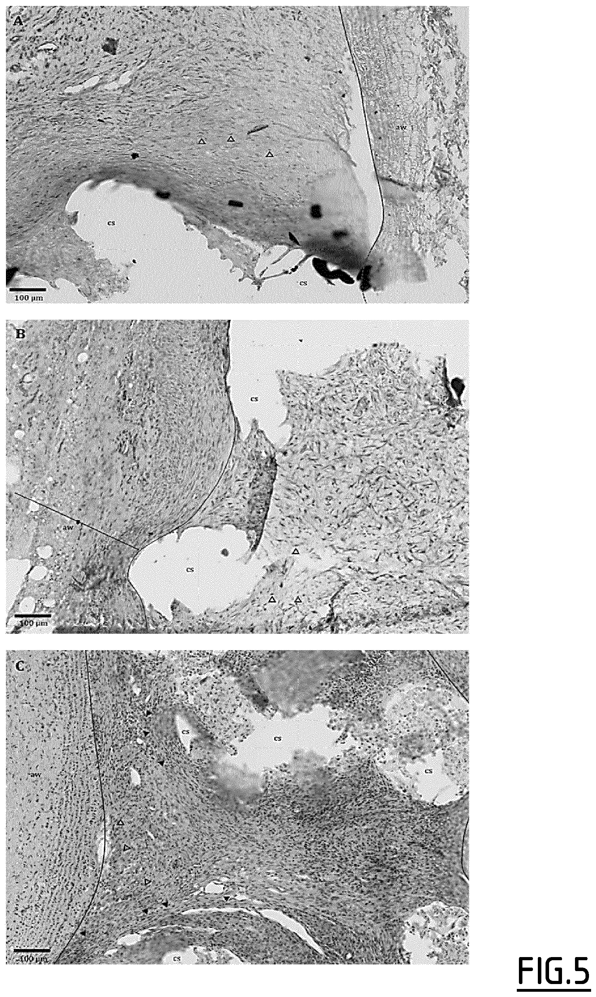

In bare coil aneurysm section (Figure 5A), as in all other groups, it was observed infiltration of cells and formation of healing tissue inside the aneurysm, that indicates a filling of the aneurysm. However, in the control group, the healing tissue presents a low cellular proliferation (the cellular nuclei appear in darker grey, as examples indicated with clear arrowheads), with a low-level organization, and a dominance of cellular matrix, including around the coil scars. In dextran-covered coil treated aneurysm (Figure 5B), almost the same results can be seen as in the untreated control, with an unorganized tissue formed, containing a low cells concentration and mostly filled with extracellular matrix.

In the fucoidan-covered coil treated aneurysm (Figure 5C), the staining reveals a more extensive cell proliferation in scar tissue than in the controls. It was also observed an orientation of cellular nuclei (flattened nuclei indicated by full arrowheads) around the coil scars, that would indicate an orientation of cells around the coils.

It follows from these experiments that the healing of the aneurysms is better in the fucoidantreated group than in the control (without fucoidan).

### Example 3: Histological index of aneurysm healing by coil type and aneurysmal area

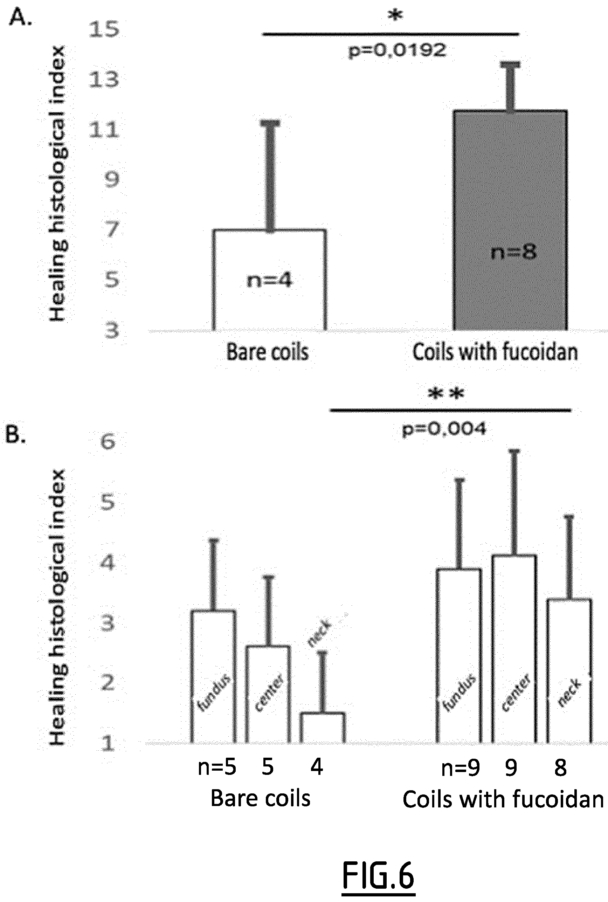

The histological index for aneurysms treated with bare coils was 7/15 versus 11/15 for coils with fucoidan with a significant difference (p=0.019) (Figure 6 A).

Considering each of the three aneurysmal zones separately, there was almost no healing at the neck for aneurysms treated with bare coils. Healing was more advanced at the center and fundus of the aneurysm than at the neck (Figure 6 B). The histological index at the neck was higher in coils with fucoidan (3.78/5) compared with bare coils (1.5/5). There was a statistically significant difference between the healing at the neck of aneurysms treated with fucoidanized versus bare coils (p=0.004, Figure 6 B).

## Claims

1. Endoprothesis for aneurysms comprising
a metallic support and
a biologically active film covalently grafted on said metallic support,
**characterized in that** said film comprises one or more polyaryl chains having a proximal end that is covalently grafted to said metallic support, and
at least one distal end that is covalently bound with a Fucoidan terminal group through a terminal functional group comprising the following group of formula (I): 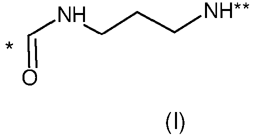
Where * represents the covalent attachment with a polyaryl chain ; and
** represents the covalent attachment with the Fucoidan terminal group.

2. Endoprothesis according to claim 1, wherein said film comprises at least one chain of formula (II) :
-***Linker**-Fucoidan (II)
Where in formula (II) :
Fucoidan represents a fucoidan polysaccharide ;
Linker represents a linear or branched polyaryl chain;
*** represents the covalent attachment of the Linker group with the metallic support and
** represents the covalent attachment of the Linker group with the Fucoidan terminal group.

3. Endoprothesis according to claim 2 wherein said Linker may be represented by the Formula (III) : 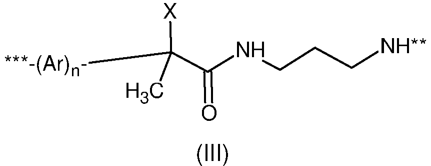
Where *** and ** are defined as in claim 2 ;
X represents H or -(Ar)ₙ- and
-(Ar)ₙ- represents a linear or branched repetition of n aryl units, with n comprised between 1 and 10 ;
wherein
Ar represents a 5 to 10 membered aryl group, this aryl group possibly comprising one or more aromatic or heteroaromatic rings, condensed or not, optionally mono- or polysubstituted;
Said optional substituents being chosen from alkyl, haloalkyl such as chloro- or fluoroalkyl, alkoxy, haloalkoxy, nitro, cyano, aldehyde, hydroxyl, ketone, carbonyl type, carboxyl, ester, ether, amine, amide, nitrile, sulfonic, halogen atoms.

4. Endoprothesis according to anyone of the preceding claims wherein the aryl group in the aryl chain and/or Ar is an optionally substituted 4-nitrophenyl of formula (IV) : 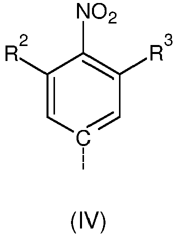 Where
--- represents a covalent bond with the metallic support;
R², R³ identical or different independently represent H or a covalent bond with another Ar unit or with the terminal functional group as defined above, provided at least one of R², R³ is a covalent bond.

5. Endoprothesis according to anyone of the preceding claims wherein said Fucoidan is a compound comprising the repetition of one or more units of formula (V) : 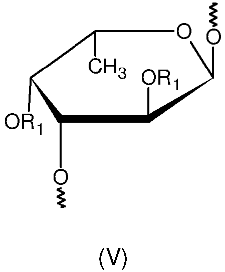
Where R₁ is chosen from H ; SO₃X where X represents H or a cation ; or a lateral sugar chosen from xylose, glucose, galactose, mannose, or a glucoronic acid ;
provided at least one of R₁ is SO₃X,
or a mixture thereof.

6. The endoprothesis according to anyone of the preceding claims wherein the Fucoidan is a mixture of average (weight) molar weight comprised between 5 and 30000 g/mol.

7. The endoprothesis according to anyone of the preceding claims which is a coil or a web.

8. The endoprothesis according to anyone of the preceding claims where the metallic support is platinum, nickel, titane, and alloys thereof.

9. Process of manufacture of the endoprothesis according to anyone of the preceding claims which comprises the following steps :
- Reductive amination of the Fucoidan ;
- Methacrylation of said reduced Fucoidan ; and
- Grafting of said functionalized Fucoidan with the metallic support by means of an aryldiazonium salt.

10. Process according to claim 9, wherein the grafting step involves reduction of the aryldiazonium salt by electrochemistry.

11. Process according to claim 9, wherein the grafting step involves reacting the metallic support with the aryldiazonium salt and the functionalized Fucoidan, in the presence of ascorbic acid.

12. Process according to anyone of claims 9 to 11, wherein the diazonium salt is of formula (VII) : 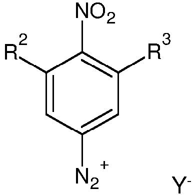 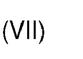 Where R², R³ identical or different independently represent H or -CH₃ or a covalent bond, and Y⁻ represents a counter ion.

13. Endoprothesis according to anyone of claims 1 to 8 for its use for the treatment of cerebral aneurysms.

14. Endoprothesis for its use according to claim 13 for preventing the recidivism of cerebral aneurysms.

## Patentansprüche

1. Endoprothese für Aneurysmen, umfassend
einen metallischen Träger und
einen biologisch aktiven Film, der kovalent auf dem metallischen Träger gepfropft ist, **dadurch gekennzeichnet, dass** der Film eine oder mehrere Polyarylketten umfasst, die ein proximales Ende aufweisen, das kovalent auf den metallischen Träger gepfropft ist, und
mindestens ein distales Ende, das kovalent mit einer terminalen Fucoidan-Gruppe durch eine terminale Funktionsgruppe verbunden ist, die die folgende Gruppe der Formel (I) umfasst: 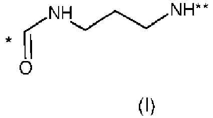
wobei * die kovalente Anbindung an eine Polyarylkette darstellt; und
** die kovalente Anbindung an die terminale Fucoidan-Gruppe darstellt.

2. Endoprothese nach Anspruch 1, wobei der Film mindestens eine Kette der Formel (II) umfasst:
-***Linker**-Fucoidan (II)
wobei in Formel (II):
Fucoidan ein Fucoidanpolysaccharid darstellt;
Linker eine lineare oder verzweigte Polyarylkette darstellt;
*** die kovalente Anbindung der Linker-Gruppe an den metallischen Träger darstellt und
** die kovalente Anbindung der Linker-Gruppe an die terminale Fucoidan-Gruppe darstellt.

3. Endoprothese nach Anspruch 2, wobei der Linker durch die Formel (III) dargestellt sein kann: 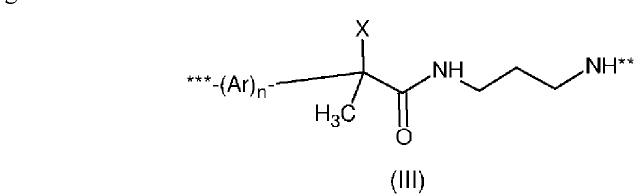
wobei *** und ** wie in Anspruch 2 definiert sind;
X für H oder -(Ar)ₙ- steht und
-(Ar)ₙ- eine lineare oder verzweigte Wiederholung von n Aryleinheiten darstellt, wobei n zwischen 1 und 10 liegt;
wobei
Ar für eine 5 bis 10-gliedrige Arylgruppe steht, wobei diese Arylgruppe möglicherweise einen oder mehrere aromatische oder heteroaromatische Ringe umfasst, die kondensiert oder nicht kondensiert und optional mono- oder polysubstituiert sind;
wobei die optionalen Substituenten aus Alkyl, Halogenalkyl wie Chlor- oder Fluoralkyl, Alkoxy, Halogenalkoxy, Nitro, Cyano, Aldehyd, Hydroxyl, Keton, Carbonyl, Carboxyl, Ester, Ether, Amin, Amid, Nitril, Sulfonsäure und Halogenatomen ausgewählt sind.

4. Endoprothese nach einem der vorhergehenden Ansprüche, wobei die Arylgruppe in der Arylkette und/oder Ar ein optional substituiertes 4-Nitrophenyl der Formel (IV) ist: wobei 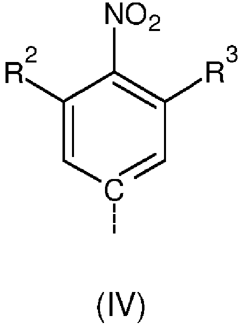
--- eine kovalente Bindung mit dem metallischen Träger darstellt;
R², R³ identisch oder unterschiedlich unabhängig voneinander H oder eine kovalente Bindung mit einer anderen Ar-Einheit oder mit der vorstehend definierten terminalen Funktionsgruppe darstellen, sofern mindestens eine von R², R³ eine kovalente Bindung ist.

5. Endoprothese nach einem der vorhergehenden Ansprüche, wobei das Fucoidan eine Verbindung ist, die die Wiederholung von einer oder mehreren Einheiten der Formel (V) umfasst: 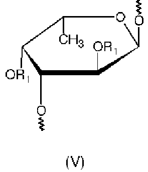
wobei R₁ aus H; SO₃X, wobei X H oder ein Kation darstellt; oder einem Seitenzucker ausgewählt ist, der aus Xylose, Glucose, Galactose, Mannose oder Glucoronsäure ausgewählt ist;
sofern mindestens eines von R₁ SO₃X oder eine Mischung davon ist.

6. Endoprothese nach einem der vorhergehenden Ansprüche, wobei das Fucoidan eine Mischung mit einem durchschnittlichen (Gewichts-)Molekulargewicht zwischen 5 und 30000 g/mol ist.

7. Endoprothese nach einem der vorhergehenden Ansprüche, bei der es sich um eine Spirale oder ein Gewebe handelt.

8. Endoprothese nach einem der vorhergehenden Ansprüche, wobei der metallische Träger aus Platin, Nickel, Titan und deren Legierungen besteht.

9. Verfahren zur Herstellung der Endoprothese nach einem der vorhergehenden Ansprüche, das die folgenden Schritte umfasst:
- reduktive Aminierung des Fucoidans;
- Methacrylierung des reduzierten Fucoidans; und
- Pfropfen des funktionalisierten Fucoidans mit dem metallischen Träger mittels eines Aryldiazoniumsalzes.

10. Verfahren nach Anspruch 9, wobei der Pfropfungsschritt die Reduktion des Aryldiazoniumsalzes durch Elektrochemie involviert.

11. Verfahren nach Anspruch 9, wobei der Pfropfungsschritt die Reaktion des metallischen Trägers mit dem Aryldiazoniumsalz und dem funktionalisierten Fucoidan in Gegenwart von Ascorbinsäure involviert.

12. Verfahren nach einem der Ansprüche 9 bis 11, wobei das Diazoniumsalz die Formel (VII) hat: 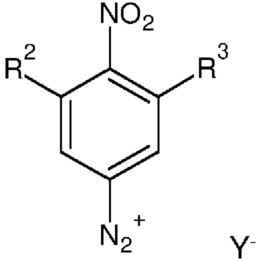 wobei R², R³ identisch oder unterschiedlich unabhängig voneinander H oder -CH₃ oder eine kovalente Bindung darstellen und Y⁻ ein Gegenion darstellt.

13. Endoprothese nach einem der Ansprüche 1 bis 8 zur Verwendung bei der Behandlung zerebraler Aneurysmen.

14. Endoprothese zur Verwendung nach Anspruch 13 zur Vorbeugung des Rezidivs zerebraler Aneurysmen.

## Revendications

1. Endoprothèse pour anévrismes comprenant
un support métallique et
un film biologiquement actif greffé de manière covalente sur ledit support,
**caractérisée en ce que** ledit film comprend une ou plusieurs chaînes polyaryles ayant une extrémité proximale greffée de manière covalente sur ledit support métallique, et au moins une extrémité distale liée de manière covalente à un groupe terminal Fucoïdane par l'intermédiaire d'un groupe fonctionnel terminal comprenant le groupe suivant de formule (I) : 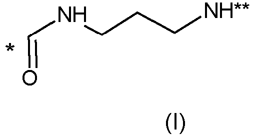
Où * représente la liaison covalente avec une chaîne polyaryle ; et
** représente la liaison covalente avec le groupe terminal fucoïdane.

2. Endoprothèse selon la revendication 1, dans laquelle ledit film comprend au moins une chaîne de formule (II) :
-***Linker**-Fucoidan (II)
Où dans la formule (II) :
Fucoidan représente un polysaccharide de fucoïdane ;
Linker représente une chaîne polyaryle linéaire ou ramifiée ;
*** représente la liaison covalente du groupe Linker avec le support métallique et
** représente la liaison covalente du groupe Linker avec le groupe terminal Fucoidan.

3. Endoprothèse selon la revendication 2, dans laquelle ledit Linker peut être représenté par la formule (III) : 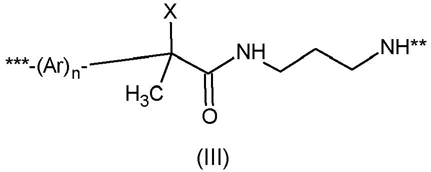
Où *** et ** sont définis comme dans la revendication 2 ;
X représente H ou -(Ar)ₙ- et
-(Ar)ₙ- représente une répétition linéaire ou ramifiée de n unités aryles, avec n compris entre 1 et 10 ;
dans lequel
Ar représente un groupe aryle de 5 à 10 atomes, ce groupe aryle pouvant comprendre un ou plusieurs cycles aromatiques ou hétéroaromatiques, condensés ou non, éventuellement mono- ou polysubstitués ;
lesdits éventuels substituants étant choisis parmi les groupes alkyle, haloalkyle tel que chloro- ou fluoroalkyle, alkoxy, haloalkoxy, nitro, cyano, aldéhyde, hydroxyle, cétone, carbonyle, carboxyle, ester, éther, amine, amide, nitrile, sulfonique, halogène.

4. Endoprothèse selon l'une quelconque des revendications précédentes, dans laquelle le groupe aryle dans la chaîne aryle et/ou Ar est un 4-nitrophényle éventuellement substitué 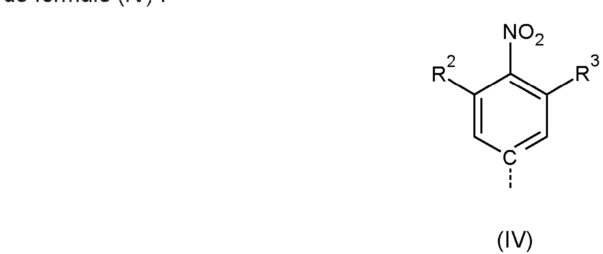 Où
- - - représente une liaison covalente avec le support métallique;
R², R³ identiques ou différents représentent indépendamment H ou une liaison covalente avec une autre unité Ar ou avec le groupe fonctionnel terminal tel que défini ci-dessus, à condition qu'au moins l'un des R², R³ soit une liaison covalente.

5. Endoprothèse selon l'une quelconque des revendications précédentes, dans laquelle ledit fucoïdane est un composé comprenant la répétition d'une ou plusieurs unités de formule (V) : 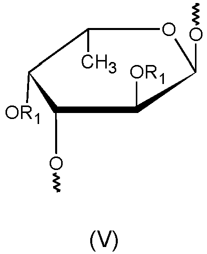 Où R₁ est choisi parmi H ; SO₃X où X représente H ou un cation ; ou un sucre latéral choisi parmi le xylose, le glucose, le galactose, le mannose ou un acide glucoronique ; à condition qu'au moins l'un des R₁ soit SO₃X, ou un mélange de ceux-ci.

6. Endoprothèse selon l'une quelconque des revendications précédentes, dans laquelle le fucoïdane est un mélange de poids molaire moyen (en poids) compris entre 5 et 30000 g/mol.

7. Endoprothèse selon l'une quelconque des revendications précédentes qui est une bobine ou une bande.

8. Endoprothèse selon l'une quelconque des revendications précédentes, dans laquelle le support métallique est du platine, du nickel, du titane et des alliages de ceux-ci

9. Procédé de fabrication de l'endoprothèse selon l'une quelconque des revendications précédentes qui comprend les étapes suivantes :
- Amination réductrice du fucoïdane ;
- Méthacrylation dudit fucoïdane réduit ; et
- Greffage dudit fucoïdane fonctionnalisé sur le support métallique au moyen d'un sel d'aryldiazonium.

10. Procédé selon la revendication 9, dans lequel l'étape de greffage implique la réduction du sel d'aryldiazonium par électrochimie.

11. Procédé selon la revendication 9, dans lequel l'étape de greffage consiste à faire réagir le support métallique avec le sel d'aryldiazonium et le fucoïdane fonctionnalisé, en présence d'acide ascorbique.

12. Procédé selon l'une quelconque des revendications 9 à 11, dans lequel le sel de diazonium est de formule (VII) : 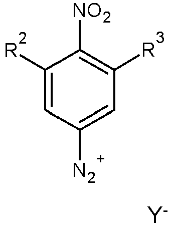 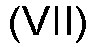 Où R², R³ identiques ou différents représentent indépendamment H ou -CH₃ ou une liaison covalente, et Y représente un contre-ion.

13. Endoprothèse selon l'une quelconque des revendications 1 à 8 pour son utilisation pour le traitement des anévrismes cérébraux.

14. Endoprothèse pour son utilisation selon la revendication 13 pour prévenir la récidive des anévrismes cérébraux.
